Europäisches Patentamt

European Patent Office

Office européen des brevets

(11) Publication number: **0 369 457**
**A2**

## EUROPEAN PATENT APPLICATION

(21) Application number: **89121294.6**

(22) Date of filing: **17.11.89**

(51) Int. Cl.⁵: **G01N 21/47, G01N 33/02**

(30) Priority: **17.11.88 DK 6434/88**

(43) Date of publication of application:
**23.05.90 Bulletin 90/21**

(84) Designated Contracting States:
**AT BE CH DE ES FR GB IT LI NL SE**

(71) Applicant: **Slagteriernes Forskningsinstitut**
**Maglegaardsvej 2**
**DK-4000 Roskilde(DK)**

(72) Inventor: **Borggaard, Claus**
**Birkedevejen 20**
**DK-4130 Viby-Sjaelland(DK)**
Inventor: **Nielsen, Torben**
**Stradjaegervej 21**
**DK-3630 Jaegerspris(DK)**

(74) Representative: **Patentanwälte Beetz sen. -**
**Beetz jun. Timpe - Siegfried -**
**Schmitt-Fumian- Mayr**
**Steinsdorfstrasse 10**
**D-8000 München 22(DE)**

(54) **Method, medium and system for the control or adjustment of optical reflection measuring devices.**

(57) The invention relates to a method for controlling or adjusting optical measuring devices comprising an optical probe and having light reflection measuring means, particularly for controlling or adjusting optical instruments used in the meat industry for determination of meat quality, such as meat colour, and/or meat classification, by introducing the probe into carcasses or cuts thereof, comprising the steps of inserting the probe into a reference medium of known light-reflective power, and controlling or adjusting the setting of the measuring device, which is characterized in that a monodisperse polymer latex is used as reference medium, preferably a polystyrene latex. This medium has an extinction which is easy to reproduce, it has a long life-time and the light-reflection value measured in the medium is practically independent of the temperature of the medium.

The invention further pertains to the polymer latex reference medium and a corresponding measurement system.

# METHOD, MEDIUM AND SYSTEM FOR THE CONTROL OR ADJUSTMENT OF OPTICAL REFLECTION MEASURING DEVICES

The present invention relates to a method and a reference medium for the control or adjustment of measuring devices comprising an optical probe, preferably instruments for meat quality determination used in the meat industry, which are provided with a probe and light reflection measuring means, which method comprises insertion of the probe into a reference medium with a known light-reflection power and control or adjustment of the setting of the instrument by means of the readings. The invention further relates to a corresponding measuring and test system.

Optical instruments are used in the meat industry for measuring the thickness of meat and fat in carcasses and cuts thereof, with the objective to determine the quality class of the individual meat pieces. Thus, e.g., the meat percentage of whole pig carcasses is determined on the basis of the weight of the carcass and some measured values of the thicknesses of meat and fat obtained on the carcass by introducing the instrument into the carcass at certain points.

To a certain extent these optical instruments may also be used to determine the colour of the meat, since pale and dark meat have different light-reflection power. Measuring the meat colour may be of great importance for deliveries to buyers who want meat of a certain pigmentation. It may also be an advantage to be able to sort out e.g. pale meat which may then be used for productions in which the pale colour is of no importance for the quality of the finished products.

The known optical instruments comprise a probe with optical means which measures the light-reflection power of the material in the immediate vicinity of the optical means. The probe may include a light emitter which illuminates the material through a lateral window provided at the side of the probe, and next to this light emitter, but screened from it, there may be a photo-detector which receives light reflected from the material. The signal from the photo-detector is an indication whether there is light or dark material in front of the window.

When measuring the meat colour and also when determining the thickness of meat and fat it is essential that the signals produced by the photo-detector are converted into precise light-reflection values, so that the meat quality may be determined precisely.

However, the light emitters and photo-detectors being used can be rather inaccurate. Their characteristics are often different, depending on the batch of production, and they are highly sensitive to changes of temperature.

Furthermore, there is a number of other natural reasons why the signal from the probes may vary considerably, e.g. the mechanical location of the components, and wear and tear on the surface of the probe. Therefore, the devices for the measuring of the light-reflection power are connected to circuits by means of which the readings may be adjusted and the variations reduced, cf. e.g. DK-B-154363.

These circuits usually comprise some potentiometers or the like, by means of which the instruments may be set to give the same readings for certain light-reflection powers. Depending on the manufacture of the instrument the adjustment may be made by inserting the probe into a hole in a white plastic plate, e.g. a block of "PERSPEX", and setting the potentiometers until the instrument shows a predetermined reading. In this way the instruments may be adjusted in proportion to a reference value, before they leave the factory, or during regular inspections.

However, the readings obtained by using instruments which are set in the known way by means of the plastic plate mentioned are not very reliable. It is a requirement that the probe is oriented in the same way relative to the plates every time a test measurement is made, and in practice this may be difficult to achieve. Moreover, the plates may easily be polluted, they may have different properties, depending on the production batch, and age and use may cause changes in the reflection properties of the plates.

It is the object of the present invention to provide a method and a medium for the control or adjustment of optical measuring devices, and particularly instruments for meat quality determination, comprising an optical probe having light reflection measuring means, by inserting the probe into a reference medium and controlling or adjusting the device in accordance with the reading or output signal of the device, which are based on a reference having a well-defined light-reflection power which may be reproduced without difficulty, to allow a direct, reliable comparison of measurements made by means of the measuring devices, even if the measurements are made at very different times and/or places, and to provide a corresponding measuring and test system.

The above object is achieved according to the claims. The dependent claims relate to preferred embodiments.

The method of the present invention for controlling or adjusting optical measuring devices comprising

2

an optical probe and having light reflection measuring means, particularly for controlling or adjusting optical instruments used in the meat industry for meat quality determination and/or classification, comprises the steps of inserting the probe into a reference medium having a known light-reflective power, and controlling or adjusting the setting of the measuring device in accordance with the readings or output signals thereof, and is characterized in that a monodisperse polymer latex is used as reference medium.

In accordance therewith, the reference medium of the present invention is a monodisperse polymer latex.

By using the medium of this invention as a reference for the control or setting of optical reflection measuring devices it is possible to obtain readings or measurement values which are very reliable, even if different instruments are used, and even if the measurements are made at different places and under different conditions, and at intervals of months or years.

The reflective power of the latex of the invention may easily be reproduced, so that the instruments are always set in proportion to a certain and constant reference. For reproduction and/or adjustment of the reflective power of a ]atex, preferably light extinction measurement is used. Thus, the light extinction of a newly manufactured or newly delivered mother latex may for instance be measured on a spectro-photometer, and the latex may be diluted with water until the desired defined extinction is obtained.

The adjusted latex may be divided into many small quantities which may be sent to the various controlling bodies, such as slaughterhouses, laboratories or foreign agencies, and these may use these samples or a quantity thereof until it becomes unsuitable for use, e.g. because of pollution. The latex medium of the invention has a stable reflective power, and therefore the controlling bodies may keep a stock of the medium which they may use over a very long period of time, e.g. more than one year, before the fixed lifetime is exceeded.

The light-reflective power of the medium according to the invention has also proved to be only slightly dependent on temperature. This is a further advantage, e.g. for controlling bodies, since the temperatures of workrooms may be very different.

Consequently, the medium of the invention is very easy to produce, standardize, and use. Polymer latices of this kind are commercialized products.

By using a monodisperse system instead of a polydisperse system several problems are avoided. Thus, in connection with polydisperse systems it is difficult to produce particles which are uniform in diameter and particle size distribution from one production batch to another, and it is also difficult to measure whether the particles are in accordance with the requirements laid down.

The particles of the monodisperse latex of the invention are of the same diameter. By means of an electron microscope it is easy to check whether the mother latex produced meets the requirements concerning a certain particle diameter.

In the individual latex the standard deviation of the diameter of the particles is very small, and thus without significance. It is usually about 1%. A monodisperse latex will e.g. only comprise particles within the range of 0.399 ± 0.004 $\mu$m.

The reflective power of a suspension of particles is highly dependent on the diameter of the particles, the refractive index of the material, the distribution of the particles etc., and for the present purpose of adjustment of optical probes the suspension in question of monodisperse particles of a polymer has proved ideal in many ways.

The physical phenomenon which is utilized in the method of the invention is a combined effect of diffusion/scattering and reflection of light by the latex particles. For the sake of convenience it is only referred to as reflection.

The latex used is preferably of a type where the diameter of the particles is within the range of 0.1 to 10 $\mu$m and particularly of 0.2 to 2 $\mu$m. In this range it is possible to obtain a reflection which has a resemblance to the reflection of meat. The density of the medium is sufficient to allow part of the light from the light emitter to be reflected to the photo-detector. The reflection does not take place at the transitional point or interface between the surface of the probe and the medium, but it occurs within an area stretching from the surface of the probe to a depth of a few millimeters into the medium. Consequently, the conditions of latex calibration are very similar to the conditions prevailing when the probe is used for measuring in meat.

A minor exceeding of the above-mentioned standard deviation of the diameter of the particles is usually tolerable, e.g. up to 2%. However, a system which is getting close to a polydisperse suspension, for instance because the particles clot, cannot be utilized with the advantages mentioned above. Therefore, it might be necessary to filter a delivered mother latex through a fine-mesh filter

A latex delivered to a controlling body may comprise stabilizers in order to keep the particles suspended or to prevent clotting for a longer period of time. If required a surface active agent may be added to the external phase.

The disperse phase of the latex consists of a synthetic polymer, e.g. a polymerisate or copolymerisate, of a styrene, vinyl or methacrylate compound. ·

The polymer is preferably a non-film-forming, hard polymer, especially a polystyrene polymer. By a non-film-forming, hard polymer it is understood a material where the surfaces during conditions of use are not apt to stick together. Many of the polymer materials known today will meet this requirement. In US-A-4,094,841 is mentioned a number of materials which are suitable for the manufacture of monodisperse latices in which the particles are not apt to stick together.

Latices which are based on polystyrene have proved to be suitable for the control and adjustment method of the invention.

In a preferred embodiment of the invention a latex is used, the disperse phase of which is polystyrene, and the continuous exterior phase is water. This latex is non-poisonous and stable, and it also gives excellent light reflection.

A latex with a dry-matter content of polymer of between 0.1 and 10 mass-% may be used, preferably between 1 and 5 mass-%. Within this range the light reflection of the latex resembles that of meat.

A latex adjusted to a predetermined reflection value may be used in the method of the invention. The reflection value is for instance determined as an extinction which is measured in a spectro-photometer under defined conditions. In a preferred embodiment of the method according to the invention a latex is used with an extinction of 0.5 to 2 at 950 nm, preferably measured in a spectro-photometer in a 2 mm quartz cuvette.

In accordance with the above, the system according to the present invention for measurement, control and adjustment of optical reflection comprises

(A) an adjustable optical measuring device comprising an optical probe which can be inserted into a medium the reflective power of which is to be measured, or into a reference medium having known light reflective power and serving for controlling or adjusting the setting of the measuring device, and having light reflection measuring means, preferably specifically adapted for use in the meat industry for meat quality determination and/or classification, and

(B) a reference medium comprising or consisting of a monodisperse polymer latex, preferably having a predetermined light reflective power, as defined above.

In this specification measuring devices and instruments with optical probes are not only to be understood as instruments with optical means placed in the probe, but also other types of instruments such as instruments with optical fibers in the probe and optical measuring means placed in the casing of the instrument.

The invention is further illustrated with more details by means of the following examples.

## Example 1

A commercial monodisperse polystyrene latex (Dow Chemicals) having a diameter of the spherical polystyrene particles of 0.399 $\mu$m, a standard deviation of the particle size of 0.06 $\mu$m and a dry-matter content of 10 mass-% is used as mother latex.

After thoroughly shaking the latex, 10 ml of the latex is pipetted and transferred to a beaker. One drop of a synthetic detergent is added to 250 ml of pure water, and 190 ml of the water is pipetted and transferred to the beaker. The diluted latex has a dry-matter content of 0.5 %. It is used as a mother solution for the production of the following dilution series (the term 'solution' being used for the sake of simplification):

4

| Mother solution | Water | Dry-matter content |
|---|---|---|
| (ml) | (ml) | (%) |
| - | - | 0.50 |
| 20.0 | 5.0 | 0.40 |
| 20.0 | 10.0 | 0.33 |
| 30.0 | 20.0 | 0.30 |
| 20.0 | 20.0 | 0.25 |
| 10.0 | 15.0 | 0.20 |
| 30.0 | 70.0 | 0.15 |

The light extinction of each of the solutions of the above dilution series is measured in a spectrophotometer in a 2 mm quartz cuvette at 950 nm. Pure water is used as reference (transmittance = 1).

A measurement of the reflection in each of the solutions is made by means of an optical probe (Radiometer No. 5607). The signal produced by the photo-diode of the probe corresponds to the reflective power of the solution. The signal provided after amplification and processing is called probe figure.

The probe is inserted into each of the solutions, and the probe figure is read during each measuring. The solutions are to be shaken vigorously, and the probe must be rinsed in water and wiped before insertion.

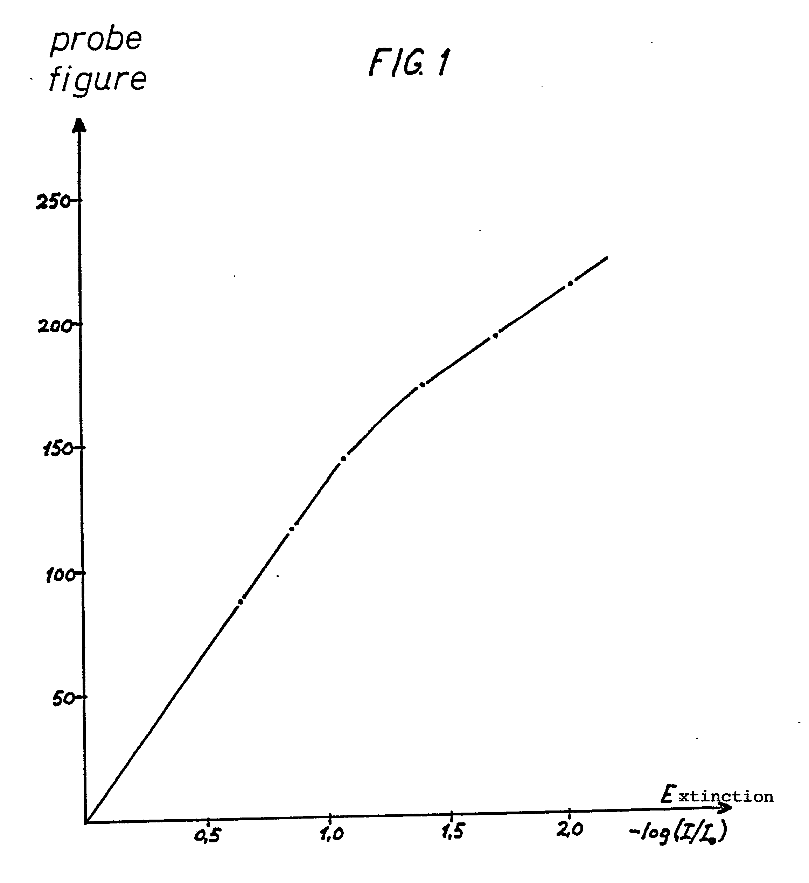

The extinction values of the solutions and the corresponding probe figures are shown in the following table. They are also illustrated in Fig. 1.

| Dry-matter content (%) | Extinction | Probe Figure |
|---|---|---|
| 0.50 | 2.02 | 213 |
| 0.40 | 1.70 | 192 |
| 0.33 | 1.39 | 179 |
| 0.30 | 1.27 | 163 |
| 0.25 | 1.07 | 143 |
| 0.20 | 0.85 | 116 |
| 0.15 | 0.64 | 87.5 |

After a fairly long time the extinction of the solutions is measured again, and the same values are found with an error of measurement of ± 0.01.

## Example 2

A mother latex corresponding to the one used in Example 1 but originating from another consignment is diluted to a dry-matter content of approx. 0.33 mass-%. The extinction of the diluted latex is measured as in Example 1, and water is added to dilute the solution until the extinction becomes 1.27.

By means of the standard solutions provided in this way, a number of probe instruments is controlled and adjusted to show the probe figure of 163. The solution may be divided into small quantities, which are sent to the control laboratories which use or sell probes of the same design.

The probe figures which are obtained by measuring in pieces of meat by means of the probe instruments adjusted in this way express in a stable, reproducible way the reflective power of the meat pieces. They are independent of the fact which probe is used or in which standard solution the device has been adjusted.

The extinction of the standard solution made above may be checked at regular intervals. When it deviates by more than the error of measurement it must be discarded, and a new quantity of a standard solution with an extinction of 1.27 should be made up.

The standard solution may also be used for the control and adjustment of other types of probe instruments or other optical probe measuring devices. Here the probe figure corresponding to the extinction of 1.27 which is the most practical value to use, will probably deviate somewhat from the probe figure of 163, but it will represent meat with the same light-reflective power as meat measured by means of the commercial probe (from Radiometer) showing the probe figure of 163.

By means of diagrams such as in Fig. 1 and corresponding figures which illustrate the probe figures of another type of probe, depending on the extinction, it is possible to correlate probe figures obtained from different types of probes, and measurements made at different places with different instruments may be compared in this way on the basis of the standard established.

## Example 3

Two different commercial monodisperse polystyrene latices (Dow Chemicals) having a dry-matter content of 10 mass-% and a standard deviation of the diameter of the spherical polystyrene particles of approx. 1 % are used:

| Diameter d | Particles/ml |
|---|---|
| ($\mu$m) | |
| Latex 1: 0.399 | 376 $\cdot$ $10^9$ |
| Latex 2: 0.822 | 43 $\cdot$ $10^9$ |

Two dilution series of the latices are made by addition of water added with a drop of a synthetic detergent.

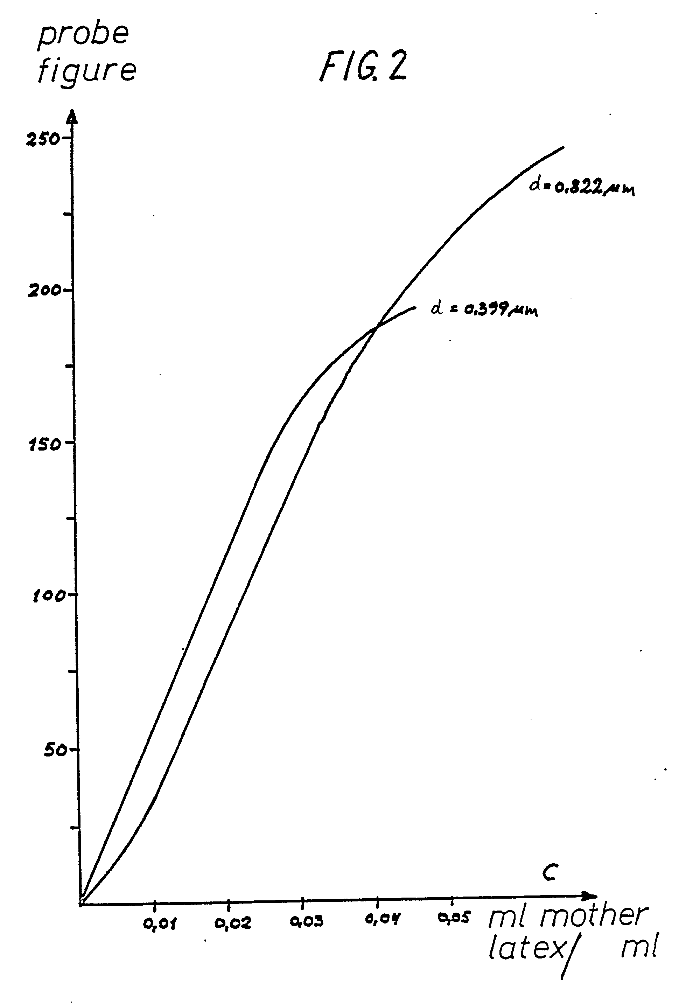

In each of the solutions of the dilution series a measurement is made with an optical probe (No. 5607). The probe figures obtained are illustrated in Fig. 2, in dependence of the degree of dilution.

As may be seen, latex 1 (d = 0.399 $\mu$m) gives the higher probe figure for a certain concentration, and both latices show a linear characteristic over a wide concentration range/measuring range of the probe. It may also be seen that the diameter of the spherical particles is essential for the probe figures obtained.

## Example 4

A dilution of a monodisperse spherical polystyrene latex with a particle diameter of 0.399 $\mu$m is used. Furthermore, another medium is used which has been made by diluting milk with water in a volume ratio of 3 : 7. The latter medium represents a polydisperse medium.

By means of various probes, which have been adjusted to show the same probe figure in a diluted monodisperse latex, measurements are made in the two media mentioned above. The measurements of the media are made at temperatures of 20 $^\circ$ and 40 $^\circ$C.

It appears from the table below that the probe figures measured in the latex medium are by and large independent of the temperature of the medium, whereas the milk medium gives temperature-dependent probe figures.

## Probe Figures

|  | Latex | | Milk | |
| --- | --- | --- | --- | --- |
| Probe No. | 20 °C | 40 °C | 20 °C | 40 °C |
| 1 | 153.9 | 150.2 | 167.0 | 154.1 |
| 2 | 153.9 | 151.5 | 167.4 | 155.4 |
| 3 | 153.9 | 152.7 | 167.3 | 157.0 |
| 4 | 153.9 | 152.4 | 166.9 | 156.0 |
| 5 | 153.9 | 151.8 | 166.3 | 156.1 |
| 6 | 153.9 | 152.4 | 167.1 | 156.1 |
| 7 | 153.9 | 151.7 | 166.7 | 155.5 |
| 8 | 153.9 | 150.7 | 165.0 | 152.1 |
| 9 | 153.9 | 151.5 | 166.1 | 156.9 |

|  | Latex | | Milk | |
| --- | --- | --- | --- | --- |
| Probe No. | 20 °C | 40 °C | 20 °C | 40 °C |
| 10 | 153.9 | 152.6 | 166.0 | 156.4 |
| 11 | 153.9 | 152.5 | 165.6 | 155.6 |
| 12 | 153.9 | 152.6 | 165.3 | 154.7 |
| 13 | 153.9 | 152.4 | 165.7 | 155.7 |
| 14 | 153.9 | 153.2 | 166.0 | 156.6 |
| 15 | 153.9 | 152.1 | 164.0 | 153.8 |
| 16 | 153.9 | 153.9 | 165.1 | 156.7 |
| 17 | 153.9 | 157.0 | 165.1 | 159.3 |
| 18 | 153.9 | 153.9 | 165.1 | 156.1 |

## Claims

1. Method for controlling or adjusting optical measuring devices comprising an optical probe and having light reflection measuring means, particularly for controlling or adjusting optical instruments used in the meat industry for meat quality determination and/or classification, comprising the steps of inserting the probe into a reference medium having a known light-reflective power, and controlling or adjusting the setting

7

of the measuring device in accordance with the readings or output signals thereof,
**characterized** in that a monodisperse polymer latex is used as reference medium.

2. The method according to claim 1,
characterized in that a latex having a particle diameter of 0.1 to 10 $\mu$m and preferably of 0.2 to 2 $\mu$m is used.

3. The method according to claims 1 and 2,
characterized in that the polymer of the latex is a non-film-forming, hard polymer, preferably a polystyrene.

4. The method according to claims 1 to 3,
characterized in that a latex having a dry-matter content of polymer of 0.1 to 10 mass-% and preferably of 1 to 5 mass-%, is used.

5. The method according to claims 1 to 4,
characterized in that a latex is used the reflective power of which has been adjusted by adjustment of the light extinction in a spectro-photometer to a predetermined extinction value, preferably an extinction of 0.5 to 2.0, measured at 950 nm.

6. Reference medium to be used for controlling or adjusting optical measuring devices comprising an optical probe and having light reflection measuring means, particularly for controlling or adjusting optical instruments used in the meat industry for meat quality determination and/or classification, comprising the steps of inserting the probe into a reference medium having a known light-reflective power, and controlling or adjusting the setting of the measuring device in accordance with the readings or output signals thereof, characterized in that it comprises or consists of a monodisperse polymer latex.

7. The reference medium according to claim 6,
characterized in that the diameter of the latex particles is of 0.1 to 10 $\mu$m and preferably 0.2 to 2 $\mu$m.

8. The reference medium according to claims 6 and 7,
characterized in that the polymer of the latex is a non-film-forming, hard polymer, preferably a polystyrene.

9. The reference medium according to claims 6 to 8,
characterized in that the latex has a dry-matter content of polymer of 0.1 to 10 mass-% and preferably 1 to 5 mass-%.

10. The reference medium according to claims 6 to 9,
characterized in that its reflective power has been adjusted by adjustment of the light-extinction in a spectro-photometer to a predetermined extinction value, preferably an extinction of 0.5 to 2.0, measured at 950 nm.

11. Use of the reference medium according to claims 6 to 10 for controlling or adjusting optical measuring devices comprising an optical probe and having light reflection measuring means, particularly for controlling or adjusting optical instruments used in the meat industry for meat quality determination and/or classification, comprising the steps of inserting the probe into a reference medium having a known light-reflective power, and controlling or adjusting the setting of the measuring device in accordance with the readings or output signals thereof.

12. System for measurement, control and adjustment of optical reflection, comprising

(A) an adjustable optical measuring device comprising an optical probe which can be inserted into a medium the reflective power of which is to be measured, or into a reference medium having known light reflective power and serving for controlling or adjusting the setting of the measuring device, and having light reflection measuring means, preferably specifically adapted for use in the meat industry for meat quality determination and/or classification, and

(B) a reference medium comprising or consisting of a monodisperse polymer latex, preferably having a predetermined light reflective power.

13. The system according to claim 12, characterized by a reference medium (B) according to claims 6 to 10.

FIG. 1

FIG. 2